(19) Europäisches Patentamt
European Patent Office
Office européen des brevets

(11) Publication number: **0 170 483**
**A2**

(12) **EUROPEAN PATENT APPLICATION**

(21) Application number: 85305222.3

(22) Date of filing: 23.07.85

(51) Int. Cl.[4]: C 07 C 67/42
C 07 C 69/86
//C07C45/54, C07C49/825, C07C45/46, C07C67/08, C07C69/157, C07C65/03, C07C51/347

(30) Priority: 24.07.84 US 633832
24.07.84 US 633831

(43) Date of publication of application: 05.02.86 Bulletin 86/6

(84) Designated Contracting States: BE DE FR GB IT NL

(71) Applicant: CELANESE CORPORATION
1211 Avenue of the Americas
New York New York 10036(US)

(72) Inventor: Davenport, Kenneth Gerald
3126 Crest Water Street
Corpus Christi Texas(US)

(74) Representative: De Minvielle-Devaux, Ian Benedict Peter et al,
CARPMAELS & RANSFORD 43, Bloomsbury Square
London WC1A 2RA(GB)

(54) **Process for producing acyloxy aromatic carboxylic acid.**

(57) Acyloxy aromatic carboxylic acids, e.g., 4-acetoxybenzoic acid, are prepared by oxidizing with oxygen an acyloxy aromatic ketone, e.g., 4-acetoxyacetophenone in the presence of transition metal ions as catalyst and a co-reductant. The acyloxy aromatic ketone may be prepared by acylating a hydroxy aromatic ketone, e.g., 4-hydroxyacetophenone, which has the effect of "masking" the hydroxyl group of the ketone in a manner necessary to effect the subsequent transition-metal catalyzed oxidation of the ketone to the acyloxy aromatic carboxyic acid.

EP 0 170 483 A2

# PROCESS FOR PRODUCING ACYLOXY AROMATIC CARBOXYLIC ACIDS

This invention relates to the catalytic oxidation of acyloxy aromatic ketones to acyloxy aromatic carboxylic acids, e.g., 4-acetoxyacetophenone to 4-acetoxybenzoic acid.

This invention also relates to an integrated process for the production of 4-acetoxybenzoic acid from phenyl acetate or phenol and an acetylating agent such as acetic acid or acetic anhydride as the starting material.

## BACKGROUND OF THE INVENTION

It is known to prepare 4-acetoxybenzoic acid (4-ABA) by reacting phenol with an alkali metal hydroxide, e.g., potassium hydroxide, to form an alkali metal phenoxide, e.g., potassium phenoxide, and reacting the phenoxide with carbon dioxide in a Kolbe-Schmitt reaction followed by acidic workup to form 4-hydroxybenzoic acid (4-HBA). The acid is then acetylated with an acetylating agent, e.g., acetic anhydride, to form the 4-ABA. A substantial disadvantage of this process is the necessity to neutralize the hydroxybenzoate salt in the course of the Kolbe-Schmitt reaction resulting in the formation of a waste alkali metal salt which must be separated and disposed of.

The preparation of hydroxy aromatic ketones by the Fries rearrangement of aromatic esters is well-known in the art.

Thus, Lewis, U.S. Patent No. 2,833,825 shows the rearrangement of phenyl or other aromatic esters to acylphenols or other hydroxy aromatic ketones using anhydrous hydrogen fluoride as catalyst. The examples of this patent are limited to the rearrangement of esters of higher fatty acids with the yields ranging from 55 to 95%.

Simons et al., Journal of the American Chemical Society, 62, 485 and 486 (1940) show the use of hydrogen fluoride as a condensing agent for various rearrangements, and at page 486, show the Fries rearrangement of phenyl acetate to obtain p-hydroxyacetophenone.

Dann and Mylius in a dissertation included as part of a series of Reports from the Institute for Applied Chemistry of the University of Erlangen, received for publication on January 7, 1954, and published in Annalen der Chemie, 587 Band, pages 1 to 15, show the rearrangement of phenyl acetate in hydrogen fluoride to 4-hydroxyacetophenone, with a maximum yield of 81% after 24 hours of reaction time, and report a yield of 92% stated to be obtained by K. Weichert as reported in Angewandte Chemie 56, 338 (1943). However, Dann and Mylius suggest that the difference in yields may be at least partly due to the previous ignoring by Weichert of the accompanying 2-hydroxy acetophenone. Dann and Mylius also report somewhat lower yields of hydroxy aromatic ketones from rearrangements in hydrogen fluoride of m-cresyl acetate, p-cresyl acetate, and guaiacol acetate.

Dann and Mylius also disclose the reaction of phenol and glacial acetic acid in the presence of hydrogen fluoride to produce 4-hydroxyacetophenone in a yield of 61.6%. This reaction may be conventionally characterized as a Friedel-Crafts acetylation of phenol with acetic acid as the acetylating agent.

Simons et al., Journal of the American Chemical Society, 61, 1795 and 1796 (1939) teach the acylation of aromatic compounds using hydrogen fluoride as a condensing agent and in Table 1 on page 1796 show the acetylation of phenol with acetic acid to produce p-hydroxyacetophenone in 40% yield.

Meussdoerffer et al., German Offenlegungsschrift 26 16 986, published October 27, 1977, and assigned to Bayer AG, disclose the acylation of phenolic compounds such as phenol itself with an acyl halide such as acetyl chloride to form hydroxy aromatic ketones.

Khandual et al., J. Indian Chem. Soc. 49, 557-560 (Eng.) (1972) as abstracted in C.A. (1972), 77, 125628g, show the oxidation of acetophenone in 95% acetic acid by manganic acetate to form benzoic acid, and formaldehyde. The oxidation of acetophenone containing ring substituents, e.g., methoxy, is also taught.

Den Hertog et al., Journal of Catalysis 6, 357-361, (1966) show the manganic acetate catalyzed oxidation of acetophenone and acetophenone containing any of various ring substituents such as methyl to benzoic acid and corresponding ring substituted benzoic acids.

Van Helden et al., Rec. Trav. Chim. 80, 57-81, (1961) show the manganese ion-catalyzed oxidations of acetophenone and various ring-substituted acetophenones to the corresponding benzoic acids and the cobalt ion-catalyzed oxidation of acetophenone to benzoic acid.

Misra et al., J. Indian Chem. Soc. 52, 1053-1055 (Eng.) (1975) as abstracted in C.A. (1976), 84, 150041n, show the vanadium catalyzed oxidation of acetophenone and acetophenones containing any of various ring substituents such as methoxy.

Kato et al., Japanese Patent No. 75 35,066 issued November 13, 1975, as abstracted in C.A. (1976), 85, 5360g; Kobayashi et al., Japanese Patent No. 67 849 issued Jan. 18, 1967, and abstracted in C.A., (1967), 66, 55236z; and Sangarah et al., Synthesis 12, 1018-1019, (1980), all show the transition metal-catalyzed oxidation of p-cresyl acetate to 4-acetoxybenzoic acid.

## SUMMARY OF THE INVENTION

In accordance with this invention, an acyloxy aromatic ketone, e.g., 4-acetoxyacetophenone (4-AAP) is oxidized with oxygen in the presence of transition metal ions and a co-reductant to produce the corresponding acyloxy aromatic carboxylic acid, e.g., 4-acetoxybenzoic acid (4-ABA). This compound may be used as is or may be hydrolyzed in acid solution ot the corresponding hydroxy aromatic carboxylic acid, e.g., 4-hydroxybenzoic acid (4-HBA).

The oxidation reaction proceeds as indicated in equation (I). In this and the following equations, the abbreviations "trans. metal" and "co-red." denote the presence of transition metal ions and a co-reductant, respectively,

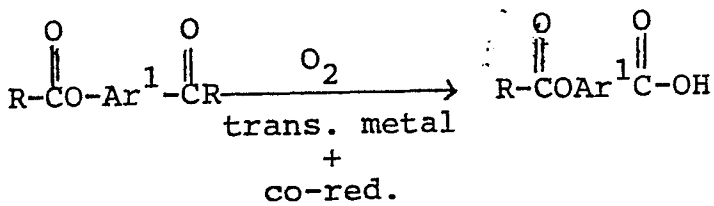


(I)

where R is a monovalent organic radical and $Ar^1$ is a divalent aromatic radical as will be more specifically defined below.

If 4-acetoxybenzoic acid is the desired product with phenyl acetate, or phenol and an acetylating agent such as acetic acid or acetic anhydride as the starting material by means of an integrated process in which the phenyl acetate, or phenol and an acetylating agent, is first converted to 4-hydroxyacetophenone (4-HAP) by a Fries Rearrangement or Friedel-Crafts acetylation respectively. The 4-HAP is then acetylated with an acetylating agent to produce 4-acetoxyacetophenone (4-AAP) which is oxidized with oxygen in the presence of transition metal ions and a co-reductant to produce 4-acetoxybenzoic acid (4-ABA). The 4-ABA may be used as is or may be hydrolyzed in acid solution to 4-hydroxybenzoic acid (4-HBA).

Although the reaction of phenol and an acetylating agent is characterized herein as a "Friedel-Crafts acetylation," no opinion as to the mechanism of reaction should be implied by this characterization.

When carrying out the process of this invention using phenyl acetate as the starting material, the initial Fries rearrangement to produce 4-hydroxyacetophenone (4-HAP) from phenyl acetate is defined by equation (II):

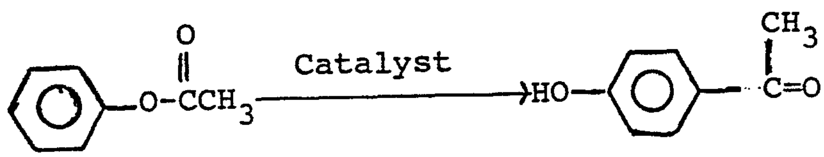


(II)

If phenol and an acetylating agent are used at the starting material, the resulting acetylation reaction to form 4-HAP is indicated by equation (III):

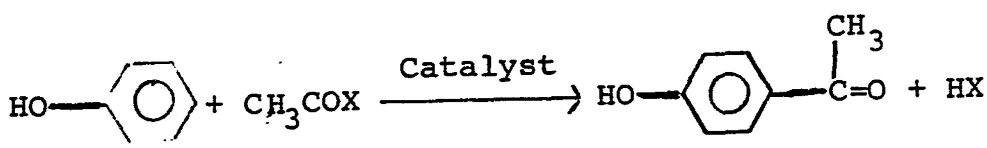


(III)

where X is the residue minus an acetyl group of compounds which are known acetylating agents. For example, X may be, hydroxy, acetoxy, or halide including fluoride, chloride, or bromide. Acetylating agents which may be used are for example, acetic acid, acetic anhydride, acetyl fluoride, acetyl chloride, and acetyl bromide.

The formation of 4-acetoxyacetophenone (4-AAP) from 4-hydroxyacetophenone (4-HAP) using acetic anhydride as the acetylating agent, proceeds as in equation (IV):

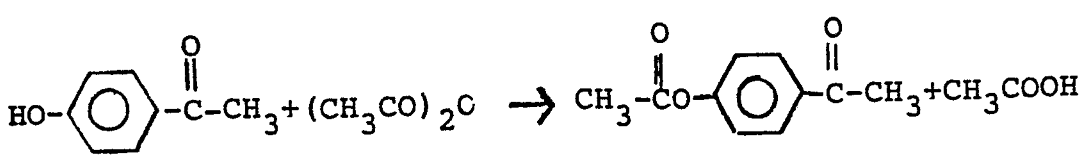

(IV)

As shown above, in the general case, the formation of 4-acetoxybenzoic acid (4-ABA) by the oxidation of 4-acetoxyacetophenone (4-AAP) proceeds as in equation (V) wherein the abbreviations "trans. metal" and "co-red." denote the presence of transition metal ions and a co-reductant, respectively.

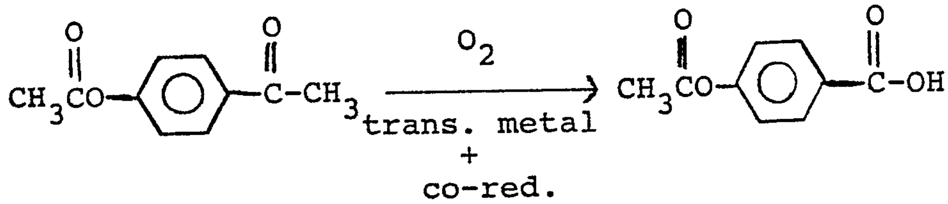


(V)

If it is desired to hydrolyze the acetoxybenzoic acid to 4-hydroxybenzoic acid (4-HBA), the reaction proceeds as in equation (VI):

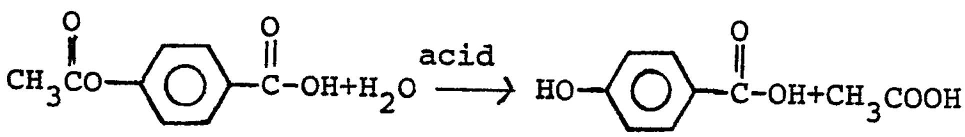


(VI)

The acylation of the hydroxy radical of the hydroxy aromatic ketone has the effect of "masking" the hydroxy radical in a manner necessary to effect the subsequent transition metal-catalyzed oxidation of the ketone group to produce the acyloxy aromatic carboxylic acid in accordance with the invention.

The formation of the acyloxy aromatic ketone from the hydroxy aromatic ketone proceeds as in equation (VII)

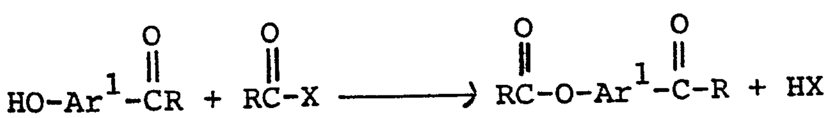

(VII)

where $Ar^1$ is a divalent aroamtic radical, R is an alkyl radical and X is the residue of an acylating agent as more fully defined below.

Where it is desired to produce 4-acetoxyacetophenone (4-AAP) from 4-hydroxyacetophenone (4-HAP) using acetic anhydride as the acetylating agent, the reaction proceeds as in equation (VIII):

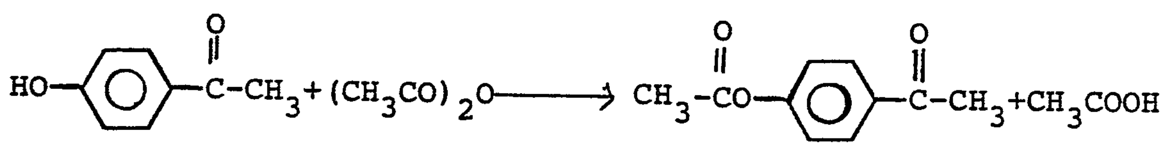 (VIII)

In the equations used to represent the reactions of this invention, $Ar^1$ is a divalent aromatic radical. The specific nature of the radical is not critical but it is preferably a radical resulting from the removal of two ring hydrogen atoms from benzene, naphthalene, or biphenyl, either unsubstituted or with ring hydrogens substituted with radicals such as methyl alkoxy or acyloxy containing 1 to 18 carbon atoms; tolyl; and appropriately masked hydroxy, amino, or sulfhydryl substitutents. $Ar^1$ is preferably 1,4-phenylene, 2,1-naphthylene, 2,6-naphthylene, 5-phenyl-1,2-phenylene, 3-phenyl-1,4-phenylene or 3-methyl-1,4-phenylene with the ketocarbon and corresponding groups occupying the first stated numbered

position of $Ar^1$ when the positions are not equivalent. Most preferably $Ar^1$ is 1,4-phenylene.

The R groups in the equations may be the same or different and are alkyl groups containing, for example 1 to 18 carbon atoms, preferably 1 to 4 carbon atms. More preferably, R is the same in all occurrences in the equations and is methyl, ethyl or propyl and most preferably methyl corresponding to the use of acetate esters and methyl ketones in the latter equations.

The hydroxy aromatic ketone used to form the acyloxy aromatic ketone may be prepared by any method known in the art. For example, it may be prepared by the Fries rearrangement of the corresponding aromatic ester as indicated by equation (IX) where $Ar^1$ and R have the definitions given herewith and Ar is an aryl radical corresponding to the definition of $Ar^1$ given herewith except that the carbon bonded to a hydroxy or acyloxy group is bonded to a hydrogen instead.

$$HAr^1O\overset{O}{\overset{\|}{C}}R \xrightarrow{\text{catalyst}} HO\text{-}Ar^1\text{-}\overset{O}{\overset{\|}{C}}R \qquad (IX)$$

Alternatively, a phenolic compound and an acylating agent may be reacted in a Friedel-Crafts acylation to form the hydroxy aromatic ketone, in accordance with the following equation:

$$HAr^1OH + R\text{-}\overset{O}{\overset{\|}{C}}\text{-}X \xrightarrow{\text{catalyst}} HO\text{-}Ar^1\text{-}\overset{O}{\overset{\|}{C}}\text{-}R + HX \qquad (X)$$

where Ar, $Ar^1$ and R have the meanings given previously and X in the equations is the residue minus the acyl group, $R-\overset{O}{\overset{\|}{C}}-$, of the compounds which are known acylating agents, such as hydroxy; acyloxy, e.g., acetoxy; and halide, e.g., fluoride, chloride, and bromide. Examples of phenolic compounds which may be employed are phenol, 1-naphthol, 2-naphthol, 2-phenylphenol, 4-phenylphenol and o-cresol. Acylating agents which may be used are for example alkanoic acids, e.g., acetic and propionic acids, alkanoic acid anhydrides, e.g., acetic and propionic anhydrides, and acyl halides, e.g., acetyl and propionyl fluorides, chlorides, and bromides.

The catalyst for both of the foregoing reactions is preferably hydrogen fluoride but any other catalyst known in the art to be effective for the Fries and Friedel-Crafts reactions may be used, e.g., aluminum chloride, zinc chloride, or boron trifluoride.

In carrying out the reaction, the aromatic ester or phenolic compound and acylating agent, catalyst, and if desired when an aromatic ester is the starting material, an additive for the reaction such as acetic anhydride or acetic acid, may be charged to a corrosion-resistant reactor and the mixture maintained at a temperature, for example, of about 20 to about 150°C for a period, for example, of about 1/2 to about 4 hours, at a pressure, for example, of about 25 to about 500 psig. If HF is used as the catalyst it may be charged as a liquid or a

gas using technologies of handling well-known to those skilled in the art. In carrying out the reaction, an inert gas such as nitrogen may be used to keep the reaction space under the desired pressure and sufficient HF in contact with the reacting liquid. An excess of HF is generally used, for example, about 7 to about 75 moles per mole of aromatic ester or phenolic compound initially present in the reaction zone. If 4-ABA is the desired product of the reaction, the starting material if a Fries rearrangement is employed will be a phenyl carboxylate, preferably phenyl acetate, while phenol and an acylating agent, preferably an acetylating agent such as acetic acid or acetic anhydride is the starting material if a Friedel-Crafts acylation is utilized. In both cases, the starting material is converted to a 4-hydroxyphenyl ketone such as 4-HAP which is in turn converted by the process of this invention to 4-ABA.

The Fries rearrangement or Friedel-Crafts reaction catalyst used in the production of 4-HAP may be hydrogen fluoride or any other catalyst known in the art to be effective for the Fries or Friedel-Crafts reaction, e.g. aluminum chloride, zinc chloride, or boron trifluoride. In carrying out the reaction, the phenyl acetate, or phenol and acetylating agent, e.g., acetic acid or acetic anhydride, catalyst, and if desired when phenyl acetate is the starting material, an additive for the reaction such as acetic anhydride or acetic acid, may be charged to a corrosion-resistant reactor and the

mixture maintained at a temperature, for example, of about 20 to about 150°C for a period, for example, of about 1/2 to about 4 hours, at a pressure, for example, of about 25 to about 500 psig. If HF is used as the catalyst it may be charged as a liquid or a gas using technologies of handling well-known to those skilled in the art. In carrying out the reaction, an inert gas such as nitrogen may be used to keep the reaction space under the desired pressure and sufficient HF in contact with the reacting liquid. An excess of HF is generally used, for example, about 7 to about 75 moles per mole of phenyl acetate or phenol initially present in the reaction zone.

The 4-acetoxyacetophenone (4-AAP) used as the starting material for the catalytic oxidation to 4-acetoxybenzoic acid may be obtained as a co-product with the 4-hydroxy-acetophenone (4-HAP) produced by the Fries rearrangement of phenyl acetate or the Friedel-Crafts acetylation of phenol, e.g., when the reaction is carried out in the presence of HF and an acid anhydride, or it may be produced from the 4-HAP by reacting the latter with an acetylating agent such as acetic anhydride, as indicated by equation (III), by contacting the 4-HAP with, for example, about 1 to 5 mols of the anhydride per mol of 4-HAP at a temperature, for example, in the range of 120 to 140°C for a period, for example, in the range of 1 to 4 hours.

The acyloxy aromatic ketone i.e., 4-acetoxyacetophenone (4-AAP) is oxidized to an acyloxy aromatic carboxylic acid as indicated by equation (I) by subjecting the ketone to a

catalytic oxidation with oxygen in the presence of transition metal ions, preferably manganese, cobalt, chromium, iron, vanadium, or molybdenum ions which are added in the form of any soluble salt, for example, a salt of the same alkanoic acid, e.g., acetic as is used as a solvent, if an alkanoic acid is so employed. A co-reductant is also present such as aldehyde, e.g., acetaldehyde; a ketone such as methyl ethyl ketone; or a halide, e.g., bromide, of an alkali metal, e.g., lithium, sodium or potassium. Preferably, the transition metal is manganese and the co-reductant is acetaldehyde. A solvent is desirably used which may be, for example, an alkanoic acid of 2 to 4 carbon atoms, mixed with its anhydride and is preferably an acetic acid/anhydride mixture. In carrying out the oxidation, oxygen may be fed to the reactor mixed with an inert gas such as nitrogen at a rate such that the amount of oxygen in the vent gas from the rector does not exceed 8%.

The reaction may be carried out by agitating the reaction mixture at a temperature, for example, of about 90 to about 225°C, preferably about 125 to 175°C, at a pressure for example, of about 100 to 1200 psig, preferably about 200 to 400 psig, for a period, for example, of about 1 to 5 hours, preferably 2 to 4 hours. The catalyst may be used in an amount, for example of about 1 to 2000 ppm based on the total reaction mass, preferably about 50 to 1200 ppm, and the co-reductant in an amount, for example of about 5 to 100 mol percent, preferably about 20 to 50 mol percent based on the 4-

AAP. The oxygen is added in a manner, as by adequate sparging, to effect a high degree of contact with and mass transfer to the liquid reaction mixture.

The transition metal ions may be added, for example, in the form of a salt of the same alkanoic acid used as the solvent, e.g., as the acetate salt when acetic acid is the solvent. In general, the reaction can be carried out to high conversion of the 4-acetoxyacetophenone to all products with a selectivity to 4-acetoxybenzoic acid, of at least 50%.

The acyloxy aromatic carboxylic acid product of equation (I) may be hydrolyzed to hydroxy aromatic carboxylic acids as shown in equation (XI):

$$R-\overset{O}{\overset{\|}{C}}OAr^1\overset{O}{\overset{\|}{C}}-OH+H_2O \xrightarrow{acid} HOAr^1C-OH+R-\overset{O}{\overset{\|}{C}}-OH + CH_3COOH \qquad (XI)$$

where $Ar^1$ and R have the definitions given previously.

If the product produced by the process is 4-ABA, and it is desired to hydrolyze such 4-ABA to 4-hydroxybenzoic acid (4-HBA), the reaction proceeds as in equation (XII):

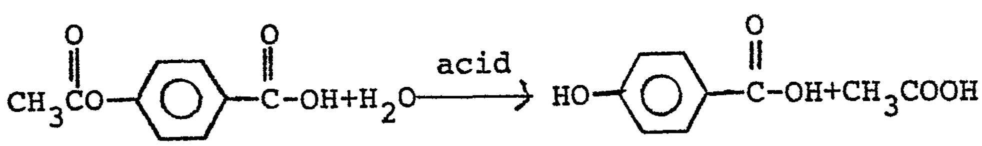


(XII)

## DESCRIPTION OF SPECIFIC EMBODIMENTS

The following examples further illustrate the invention.

### Example 1

This example illustrates the preparation of 4-hydroxyacetophenone by the Fries rearrangement of phenyl acetate using hydrogen fluoride as catalyst.

To a 300 cc Hastelloy C autoclave was charged 40.8 g (0.3 mol) of phenyl acetate. The autoclave was sealed, immersed in a dry ice/isopropanol bath and cooled internally to -45°C, and evacuated to ca. 100 Torr. Addition of 120 g (6.0 mol) of anhydrous hydrogen fluoride was performed in a manner such as that the internal temperature of the autoclave did not exceed 0°C. The internal pressure of the reactor was then adjusted to 0 psig with nitrogen. The contents of the autoclave were stirred and heated to 75°C for 1 h. The hydrogen fluoride was vented over a 45 min period at ca. 45°C. The mixture was poured onto 25 g of ice and neutralized with 45% potassium hydroxide solution. The aqueous mixture was extracted with ethyl acetate. The organic fraction was then dried over anhydrous magnesium sulfate, filtered, and the solvent was removed on a rotary evaporator to yield 44.0 g of a dark green solid corresponding to 99.9% conversion of phenyl acetate and 94.3% selectivity to 4-hydroxyacetophenone.

### Example 2

This example illustrates the preparation of 4-hydroxyacetophenone by the Fries rearrangement of phenyl acetate using

hydrogen fluoride as catalyst with acetic anhydride as additive.

To a 300 cc Hastelloy C autocalve were added 30.6 grams (0.3 mole) of acetic anhydride. The autoclave was cooled to -50°C and evacuated to 5 Torr whereupon 120 g (6.0 mole) of anhydrous hydrogen fluoride was transferred from a cylinder to the autoclave. After the transfer of hydrogen fluoride was completed, the internal temperature and the internal pressure of the autoclave was adjusted to -50°C and 0 psig using nitrogen, respectively. To the stirred autoclave was added 81.6 g (0.6 mol) of phenyl acetate at such a rate that the temperature of the mixture did not exceed -23°C. Upon completion of phenyl acetate addition, the contents were warmed to 50°C and stirred for 3 h during which time a pressure of ca. 40 psig was generated. At the end of the run, the hydrogen fluoride was vented through a caustic scrubber and the contents of the autoclave were poured onto ca. 30 g of ice. The pH of the mixture was then extracted with 75 ml of ethyl acetate (3x). The organic solution was dried over anhydrous magnesium sulfate, filtered, and the solvent was removed using a rotary evaporator.

The reaction proceeded with 98.1% conversion of phenyl acetate and with the following selectivities: phenol 1%; 4-hydroxyacetophenone (4-HAP) 82.3%; 2-hydroxyacetophenone (2-HAP) 4.3%; 3-hydroxyacetophenone (3-HAP) 0.1%; 4-acetoxyacetophenone (4-AAP) 3.8%; and 4-(4'-hydroxyphenyl)-acetophenone (HPAP) 0.4%.

Example 3

This example describes the formation of 4-hydroxyacetophenone by the Fries rearrangement of phenyl acetate using hydrogen fluoride as catalyst and acetic acid as additive.

The procedure for Example 2 was repeated except that 18 grams (0.3 mole) of acetic acid rather than acetic anhydride were charged to the reactor before it was cooled and charged with the hydrogen fluoride. A conversion of 99.0% of phenyl acetate was obtained with the following selectivities: phenol 3.3%; acetic acid 0.8%; 4-HAP 80.8%; 3-HAP 0; 2-HAP 5.8%; 4-AAP 0.3%; and HPAP 0.3%.

Example 4

This example illustrates the preparation of 4-hydroxyacetophenone (4-HAP) by the Friedel-Crafts acetylation of phenol with acetic acid as the acetylating agent.

Phenol (9.4 g, 0.1 moles) and acetic acid (12.0 g, 0.2 moles) were charged to a 300 ml Hastelloy C autoclave at room temperature. The reactor was evacuated and cooled to -20°C. HF (100 g, 5 moles) was then transferred into the reactor. The reactor was heated to 80°C and maintained for 1 hour at reaction temperature. At the end of the reaction the reactor was cooled to 20°C and the excess HF was vented to a KOH scrubber. Ethyl acetate was added to the contents of the reactor. The mixture was then neutralized with 45% aqueous KOH. The resulting organic phase was separated, dried over $MgSO_4$ and

evaporated to afford a yellow solid which contained 13.1 g (0.096 moles) of 4-HAP.

Example 5

This example illustrates the preparation of 4-hydroxyacetophenone by the Friedel-Crafts acetylation of phenol with acetic anhydride as the acetylating agent.

To a 300 cc Hastelloy C autoclave cooled to -20 °C and evacuated to 50 Torr was added 150 g (7.5 mole) of anhydrous hydrogen fluoride. The content of the autoclave was warmed to 50°C resulting in an internal pressure of 25 psig. A solution of 23.5 g (0.25 mol) of phenol and 25.5 g (0.25 mol) of acetic anhydride was added to the autoclave over a 3 minute period causing the pressure to drop to 14 psig. The solution was stirred for 1 hour at 50°C whereupon the hydrogen fluoride was vented at the reaction temperature. The contents of the autoclave were poured onto ice and the aqueous phase was adjusted to pH - 6.0 with a 45% solution of potassium hydroxide. The aqueous phase was extracted with 75 mL of ethyl acetate (3x); the organic fractions were combined, dried over anhydrous magnesium sulfate, and filtered. The reaction proceeded with 99% conversion based on phenol and 95% selectivity to 4-hydroxyacetophenone.

The following example illustrates the reaction of 4-hydroxyacetophenone (4-HAP) with acetic anhydride to form 4-acetoxyacetophenone (4-AAP).

Example 6

A solution of 136.2 g (1.0 mol) of 4-hydroxyacetophenone and 400 mL of acetic anhydride was heated at reflux for 3 h under a nitrogen atmosphere. The acetic acid and acetic anhydride was distilled overhead in vacuo (39-41 °C, 2.6 mm Hg). The remaining oil was then distilled in vacuo (132-134 °C, 2.0 mm Hg) to yield 169.7 g (95.2%) of white crystals identified as 4-acetoxyacetophenone.

Examples 7 to 10 illustrate the oxidation of 4-acetoxyacetophenone (4-AAP) to 4-acetoxybenzoic acid (4-ABA).

Example 7

A 300 cc Hastelloy C autoclave was charged with 17.8 g (0.1 mol) of 4-acetoxyacetophenone (4-AAP), 0.25 g of manganese (II) acetate tetrahydrate, 1.0 g of acetaldehyde, 25 g of acetic anhydride, and 125 g of acetic acid. An oxygen/nitrogen mixture was sparged into the autoclave at 1000 cc/min such that 5% oxygen was maintained in the vent. A 10% acetaldehyde in acetic acid solution was fed at a rate of 3.0 cc/h. The reaction ws run at 150°C and 100 psig for 3 h. using a stirring speed of 1000 rpm whereupon the acetic acid and acetic anhydride were removed on a rotary evaporator to yield orange crystals. The crystals were dissolved in ca. 150 mL of ethyl acetate and the solution was extracted with 100 mL of water (3x). The organic phase was dried over anhydrous magnesium sulfate, filtered, and the solvent was removed on a rotary evaporator to yield 4-acetoxybenzoic acid (4-ABA) at a conversion of 92.7% based on 4-AAP and with 55.1 % selectivity to 4-ABA.

Example 8

The procedure of Example 7 was followed except that the temperature of reaction was 125°C and the period of reaction was 5 hours. A conversion of 97.9% of 4-AAP was obtained with a selectivity to 4-ABA of 53.1%.

Example 9

The procedure of Example 7 was followed except that the temperature of reaction was 175°C. The conversion of 4-AAP was 89.4% with a selectivity to 4-ABA of 44.2%.

Example 10

The procedure of Example 7 was followed except that the pressure of reaction was 200 psig. The 4-AAP conversion was 97.8% with a selectivity to 4-ABA of 51.6%.

The procedures of the examples may also be used to prepare 4-acetoxy-3-methylbenzoic acid from o-cresyl acetate or o-cresol and acetic acid; 1-acetoxy-2-naphthoic acid from 1-naphthyl acetate or 1-naphthol and acetic acid; 6-acetoxy-2-naphthoic acid from 2-naphthyl acetate or 2-naphthol and acetic acid; 2-acetoxy-5-phenylbenzoic acid from 4-phenylphenyl acetate or 4-phenylphenol and acetic acid; and 4-acetoxy-3-phenylbenzoic acid from 2-phenylphenyl acetate or 2-phenylphenol and acetic acid.

The acyloxy aromatic carboxylic acid of this invention, e.g., 4-ABA may be utilized as monomers in the preparation of

polymers capable of forming an anisotropic melt phase and suitable for being formed into shaped articles such as molded articles, fibers and films, as shown, for example in U.S. Patents Nos. 4,339,375; 4,341,688; 4,351,918; and 4,355,132.

The acyloxy aromatic carboxylic acids of this invention, e.g., 4-ABA, may also be hydrolyzed to form the corresponding hydroxy aromatic carboxylic acids, e.g., 4-hydroxybenzoic acid (4-HBA) which has many uses in organic syntheses, and as an intermediate for preservatives, dyes and fungicides. The following example illustrates this process.

Example 11

A solution of 4.5 g (0.025 mole) of 4-acetoxybenzoic acid, 25 g of dimethoxyethane, 25 g of water, and 6.1 g of concentrated sulfuric acid was heated at reflux for 2 hours under a nitrogen atmosphere. The solution was cooled, saturated with sodium chloride, and extracted with 75 mL of ethyl acetate (3x). The organic fractions were combined, dried over anhydrous magnesium sulfate, and filtered. Rotary evaporation afforded a substantially quantitative yield of 4-hydroxybenzoic acid based on the 4-acetoxybenzoic acid.

CLAIMS

1. A process comprising oxidizing an acyloxy aromatic ketone with oxygen in the presence of transition metal ions and a co-reductant to form an acyloxy aromatic carboxylic acid.

2. The process of claim 1 wherein said ketone is 4-acetoxyacetophenone, said transition metal ions are manganese ions, said co-reductant is acetaldehyde, and said product is 4-acetoxybenzoic acid.

3. The process of claim 2 wherein said 4-acetoxy-benzoic acid is hydrolyzed to 4-hydroxybenzoic acid.

4. The process of claim 1 wherein the process additionally comprises reacting a hydroxy aromatic ketone with a carboxylic acid anhydride to form the acyloxy aromtic ketone.

5. The process of claim 4 wherein said hydroxy aromatic ketone is 4-hydroxyacetophenone, said acyloxy aromatic ketone is 4-acetoxyacetophenone, and said acyloxy aromatic carboxylic acid is 4-acetoxybenzoic acid.

6. The process of claim 4 wherein the process additionally comprises contacting phenyl acetate or phenol and an acetylating agent with a Fries - Friedel-Crafts reaction catalyst to form 4-hydroxyacetophenone, and acetylating the latter with an acetylating agent to form 4-acetoxyacetophenone.

7. The process of claim 6 wherein said Fries-Friedel-Crafts reaction catalyst is hydrogen fluoride.

8. The process of claim 7 wherein said 4-acetoxybenzoic acid is hydrolyzed to form 4-hydroxybenzoic acid.

9. The process of claim 7 wherein the Fries rearrangement of phenyl acetate is employed to produce 4-hydroxyacetophenone as the first step in the process.

10. The process of claim 7 wherein the Friedel-Crafts acetylation of phenol with an acetylating agent is employed to produce 4-hydroxyacetopheone as the first step in the process.

11. The process of claim 10 wherein both acetylating agents are acetic anhydride.

12. The process of claim 11 wherein said transition metal ions are manganese ions.

13. The process of claim 12 wherein said co-reductant is acetaldehyde.